# EUROPEAN PATENT SPECIFICATION

(11) **EP 1 162 935 B1**
(45) Date of publication and mention of the grant of the patent: **03.09.2003**
(21) Application number: 01901312.7
(22) Date of filing: 17.01.2001
(51) Int. Cl.: A61K 7/00, A61K 7/06, A61K 7/075

(54) **USE OF ZEOLITE IN COSMETIC PREPARATIONS FOR SKIN AND HAIR CARE**
VERWENDUNG VON ZEOLITH IN KOSMETISCHEN HAAR- UND HAUTPFLEGEMITTELN
PREPARATIONS COSMETIQUES POUR LA PEAU ET LES SOINS DE CHEVEUX

(30) Priority: 18.01.2000 HR 20000029
(43) Date of publication of application: 19.12.2001
(73) Proprietor: Mustapic, Marina, 10000 Zagreb (HR)
(72) Inventor: Mustapic, Marina, 10000 Zagreb (HR)
(74) Representative: Bonini, Ercole
(86) International application number: HR0100001
(87) International publication number: WO01052793

(56) References cited:
- EP-A- 0 950 400
- EP-A- 1 101 485
- WO-A-86/05389
- PATENT ABSTRACTS OF JAPAN vol. 1999, no. 08, 30 June 1999 (1999-06-30) & JP 11 060460 A (KAO CORP), 2 March 1999 (1999-03-02)
- PATENT ABSTRACTS OF JAPAN vol. 010, no. 017 (C-324), 23 January 1986 (1986-01-23) & JP 60 174707 A (KANEBO KK), 9 September 1985 (1985-09-09)

## Description

### 1. THE FIELD THAT THE INVENTION REFERS TO

The invention refers to products for medical, dental and hygienic purposes, precisely, to cosmetic and similar hygienic products for skin and hair care.

According to the International patent classification (7^{th} edition) this invention is placed in section A - Human necessities
A 61 K 7/00 Cosmetics or similar toilet preparations

### 2. TECHNICAL PROBLEM

With the cosmetic products as per this invention, the entire skin and hair care is achieved by the use of natural substances which do not cause allergic reactions or other side - effects/contra indications as known up to the present, and which are absolutely independent on the type of skin or hair.

### 3. BACKGROUND ART

Such or similar patent protected cosmetic preparations are unknown to the applicant/inventor.
*Patent Abstracts of Japan vol. 1999, no. 08, 30 June 1999 (1999-06-30) & JP 11 060460 A (KAO CORP), 2 March 1999 (1999-03-02) discloses a cosmetic composition which contains activated zeolite and powder of a Guinea pepper.*
*Document EP-A-0 950 400 (KAO CORP) 20 October 1999 (1999-10-20) discloses a non-aqueous cosmetic composition which comprises activated zeolite together with other ingredients such as polyhydric alcohol and compound with high molecular weight*.

### 4. DISCLOSURE OF THE INVENTION ESSENCE

Cosmetic preparations for skin and hair care according to the invention are products in the form of creams, (e.g. day or night creams,... ), milks (e.g. body milk), oily emulsions (e.g. oily body emulsion), protective emulsions (e.g. sun protective emulsion), shampoos (e.g. hair shampoo).

The basic ingredient to all products is zeolit. Common ingredients to all products are kuzu root and ginger. Dependent on kind and purpose, other ingredients to the particular preparations are:
a) almond oil,
b) jojoba oil,
c) marigold (Calendula Officinalis),
d) propolis,
e) avocado oil,
f) gavez,
g) olive oil,
h) cantaraon (Hypericum Perforatum),
i) cocoa butter,
j) yarrow (Achillea Millefolium),
k) walnut,
l) beeswax,
m) water,
n) plant oil extracts and other less essential ingredients.

### 5. DESCRIPTION OF THE PREFERRED EMBODIMENT

The substances in appropriate form (powder, oil, ... ) are heated separately and/or as an adequate mixture to a certain temperature depending on the kind of the product. This is followed by the process of the substances and/or mixture consolidation and mixing in the course of cooling.

Adequately treated zeolit is added into the cooled mixture and the process of product finalization is ended by this.

The basic substance to all preparations is zeolit in the quantity of 0,1 - 70 % wt, more preferably 3 - 50 % wt, and most preferably 5 - 25 % wt.

The common substance to all preparations is kuzu root in the quantity of 0,5 - 50 % wt, more preferably 1 - 20 % wt, and most preferably 1,5 - 2 % wt.

The second common substance to all products is ginger in the quantity of 0,2 - 30 % wt, more preferably 0,3 - 10 % wt, and most preferably 0,5 - 8 % wt.

### 6. THE INVENTION APPLICATION

The cosmetic preparations as per invention are applicable to all types of skin and hair according to the specific application for each particular product. All cosmetic preparations have undergone the statutory regulated tests and are provided with analytic reports issued by authorized institutions.

All the substances being of natural origin and their essential properties being not reduced and/or changed during the process of manufacturing, the patented preparations do not cause allergic reactions known up to present, nor any other side effects/contra indications.

## Claims

1. A cosmetic preparation comprising tribo-mechanically activated zeolite, ginger and kuzu root.

2. The cosmetic preparation according to claim 1, **characterized in that** the components are present in preparation in the following amounts:
- tribo-mechanically activated zeolite 0,1-70,0% wt
- ginger 0,2-30,0% wt
- kuzu root 0,5-50,0% wt.

3. The cosmetic preparation according to claim 1, **characterized in that** the components are present in preparation in the following amounts:
- tribo-mechanically activated zeolite 3,0-50,0% wt
- ginger 0,3-10,0% wt
- kuzu root 1.0-20,0% wt.

4. The cosmetic preparation according to claim 1, **characterized in that** the components are present in preparation in the following amounts:
- tribo-mechanically activated zeolite 5,0-25,0% wt
- ginger 0,5-8,0% wt
- kuzu root 1,5-2,0% wt.

5. The cosmetic preparation according to any of the preceding claims, **characterized in that** it comprises one or more of the following substances like almond oil, jojoba oil, marigold, propolis, avocado oil, gavez, olive oil, cantaraon, cocoa butter, yarrow, walnut, beeswax, water, plant oil extracts.

6. Use of cosmetic preparation according to any of the preceding claims, wherein the product are in the form of creams, milks, oily emulsions, protective emulsions or shampoos.

7. Use of cosmetic preparation according to any of the preceding claims for skin treatment and/or hair treatment.

## Patentansprüche

1. Ein kosmetisches Präparat mit tribomechanisch aktiviertem Zeolith, Ingwer und Kuzuwurzel.

2. Das kosmetische Präparat gemäß Patentanspruch 1), **dadurch gekennzeichnet, dass** die Bestandteile in den folgenden Mengen im Präparat enthalten sind:
- tribomechanisch aktivierter Zeolith 0,1 - 70,0 % Gew.-Ant.
- Ingwer 0,2 - 30,0 % Gew.-Ant.
- Kuzu-Wurzel 0,5 - 50,0 % Gew.-Ant.

3. Das kosmetische Präparat gemäß Patentanspruch 1), **dadurch gekennzeichnet, dass** die Bestandteile in den folgenden Mengen im Präparat enthalten sind:
- tribomechanisch aktivierter Zeolith 3,0 - 50,0 % Gew.-Ant.
- Ingwer 0,3 - 10,0 % Gew.-Ant.
- Kuzu-Wurzel 1,0 - 20,0 % Gew.-Ant.

4. Das kosmetische Präparat gemäß Patentanspruch 1), **dadurch gekennzeichnet, dass** die Bestandteile in den folgenden Mengen im Präparat enthalten sind:
- tribomechanisch aktivierter Zeolith 5,0 - 25,0 % Gew.-Ant.
- Ingwer 0,5 - 8,0 % Gew.-Ant.
- Kuzu-Wurzel 1,5 - 2,0 % Gew.-Ant.

5. Das kosmetische Präparat gemäß jeglichem der vorstehenden Patentansprüche, **dadurch gekennzeichnet, dass** es eine oder mehrere der nachstehenden Substanzen enthält, wie Mandelöl, Jojobaöl, Ringelblume, Propolis, Avocadoöl, Symphytum officinalis, Olivenöl, Hypericum Perforatum, Kakaobutter, Achillea Millefolium, Walnuss, Bienenwachs, Wasser, Pflanzenölextrakte.

6. Verwendung des kosmetischen Präparats gemäß jeglichem der vorstehenden Patentansprüche, wobei es sich bei den Produkten um Creme, Milch, ölige Emulsion, Schutzemulsion oder Shampoo handeln kann.

7. Verwendung des kosmetischen Präparats gemäß jeglichem der vorstehenden Patentansprüche für die Pflege von Haut und/oder Haar.

## Revendications

1. Une préparation cosmétique comprenant du zéolithe activé tribomécaniquement, gingembre et racine de Kuzu.

2. La préparation cosmétique selon la revendication 1), **caractérisée en ce que** les composants sont présents dans la préparation dans les quantités suivantes:
- zéolithe activée tribomécaniquement 0,1-70,0% poids
- gingembre 0,2-30,0% piods
- racine de Kuzu 0,5-50,0% poids

3. La préparation cosmétique selon la revendication 1), **caractérisée en ce que** les composants sont présents dans la préparation dans les quantités suivantes:
- zéolithe activée tribomécaniquement 3,0-50,0% poids
- gingembre 0,3-10,0% poids
- racine de Kuzu 1,0-20,0% poids

4. La préparation cosmétique selon la revendication 1), **caractérisée en ce que** les composants sont présents dans la préparation dans les quantités suivantes:
- zéolithe activée tribomécaniquement 5,0-25,0% poids
- gingembre 0,5-8,0% poids
- racine de Kuzu 1,5-2,0% poids

5. La préparation cosmétique selon une quelconque des revendications précédentes, **caractérisée en ce qu'**elle comprend une ou plusieurs des substances suivantes comme l'huile d'amande, huile de jojoba, souci, propolis, huile d'avocat, consoude officinale, huile d'olive, Hypericum Perforatum, beurre de cacao, achillée millefeuille, noix, cire d'abeille, eau, extracts d'huile de plante.

6. Emploi de la préparation cosmétique selon une quelconque des revendications précédentes, où les produits sont sous forme de crèmes, laits, émulsions de type huileux, émulsions protectrices ou shampoings.

7. Emploi de la préparation cosmétique selon une quelconque des revendications précédentes pour le traitement de la peau et/ou le traitement des cheveux.
